# EUROPÄISCHE PATENTSCHRIFT

(11) **EP 1 690 860 B1**
(45) Veröffentlichungstag und Bekanntmachung des Hinweises auf die Patenterteilung: **13.08.2014**
(21) Anmeldenummer: 06114976.1
(22) Anmeldetag: 18.06.2003
(51) Int. Cl.: C07D 313/00, C07D 315/00, C07D 311/94

(54) **Verfahren zur Herstellung von 11(12)-Pentadecen-15-oliden und von 1-Hydroperoxy-16-oxabicyclo[10.4.0]hexadecan**
Process for the preparation of 11(12)-pentadecen-15-olides and of 1-hydroperoxy-16-oxabicyclo[10.4.0]hexadecane
Procédé de préparation de 11(12)-pentadécen-15-olides et de 1-hydroperoxy-16-oxabicyclo[10.4.0]héxadécane

(30) Priorität: 19.06.2002 DE 10227483
(43) Veröffentlichungstag der Anmeldung: 16.08.2006
(62) Teilanmeldung aus: 03013771.5
(73) Patentinhaber: Symrise AG, 37603 Holzminden (DE)
(72) Erfinder: Esser, Dr. Peter, 37639 Bevern (DE); Koch, Oskar, 37079 Göttingen (DE); Marks, Werner, 37647 Brevörde (DE); Klein, Jared, Summerville, SC 29485 (US)
(74) Vertreter: Eisenführ Speiser

(56) Entgegenhaltungen:
- EP-A- 0 424 787
- EP-A- 0 503 312
- EP-A- 0 862 911
- WO-A-97/32948
- FR-A- 2 043 784
- OGIBIN Y N ET AL.: "Ring expansion reaction of 1-hydroperoxy-16- oxabicyclo[10.4.0]hexadecane catalyzed by copper ions: use in the synthesis of 15-pentadecanolide" RUSSIAN CHEMICAL BULLETIN., Bd. 47, Nr. 6, Juni 1998 (1998-06), Seiten 1166-9, XP002254763

## Beschreibung

Die Erfindung betrifft ein Herstellungsverfahren für 1-Hydroperoxy-16-oxabicyclo[10.4.0]hexadecan (DDP-OOH), gemäß den beigefügten Patentansprüchen.

Die makrocyclischen Lactone 11-Pentadecen-15-olid (=15-Hydroxypentadec-11-en-säurelacton) und 12-Pentadecen-15-olid (=15-Hydroxypentadec-12-ensäurelacton) sowie deren Gemische (11(12)-Pentadecen-15-olide) sind bekannte Moschusriechstoffe. Dabei sind sowohl die jeweiligen (E)- als auch (Z)-Formen als auch deren Gemische geruchlich interessant. In EP-A 424 787 sind die geruchlichen Eigenschaften dieser Substanzen beschrieben. Es ist ebenfalls hinreichend bekannt, dass aus den 11(12)-Pentadecen-15-oliden mittels Hydrierung 15-Pentadecanolid (15-Hydroxypentadecansäurelacton) erhalten werden kann, welches ebenfalls als Moschusriechstoff verwendet wird.

Die Herstellung von 11(12)-Pentadecen-15-oliden ist an sich bekannt. Die heute wichtigsten Herstellungsverfahren gehen von 13-Oxabicyclo[10.4.0]hexadec-1(12)-en (DDP) aus. Mittels säurekatalysierter Addition von Wasserstoffperoxid an DDP wird 1-Hydroperoxy-16-oxabicyclo[10.4.0]hexadecan (DDP-OOH) erhalten. Als entscheidender Schritt in der Synthese zu den 11(12)-Pentadecen-15-oliden ist die Spaltung des DDP-OOH unter Bildung des makrocyclischen Rings zu sehen. Diese Spaltung wird meist in Gegenwart von Katalysatoren wie Cu(OAc)₂ und gegebenenfalls FeSO₄ durchgeführt. Wird diese Reaktionsstufe rein thermisch durchgeführt, so enthält das Reaktionsprodukt erhebliche Mengen der gesättigten Verbindung 15-Pentadecanolid, welche zwar ein Moschusriechstoff ist, jedoch andere geruchliche Eigenschaften als die 11(12)-Pentadecen-15-olide aufweist und daher nur in möglichst geringen Mengen entstehen sollte. Darüber hinaus ist bei der rein thermischen Spaltung eine hohe Rückstandsbildung (z.B. Destillationssumpf) nachteilig.

DDP wird üblicherweise durch säurekatalysierte Cyclisierung von 2-(3-Hydroxypropyl)-1-cyclododecanon (OCP) unter Wasserabspaltung erhalten, welches wiederum durch radikalische Addition von Allylalkohol an Cyclododecanon synthetisiert werden kann (z.B. in DE-OS 2 136 496).

Der Prozess zur Herstellung der 11(12)-Pentadecen-15-olide kann durch das folgende Schema verdeutlicht werden:

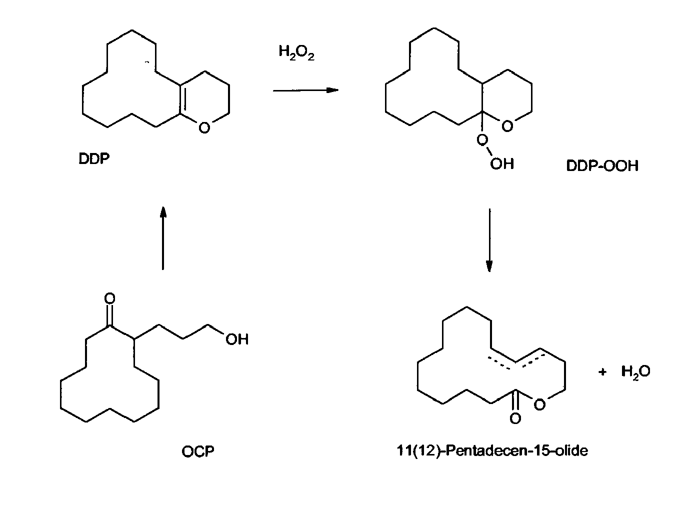

In EP-A 424 787 wurde OCP in 4,6 Gewichtequivalenten Eisessig bei Umgebungstemperatur homogenisiert, eine kalte 25 %ige wässrige Lösung von Schwefelsäure (etwa 51 mol% (etwa 21 Gew.%) bezogen auf OCP) zugegeben und die Reaktionsmischung anschließend auf 0°C abgekühlt. Danach wurden 1,65 molare Equivalente an Wasserstoffperoxid (70%ige Lösung) zugegeben, wobei die Temperatur auf 7°C anstieg. Nach einer kurzen Nachreaktionszeit wurde der gebildete Feststoff (DDP-OOH) abfiltriert, dieser mit Wasser und wässriger NaHCO₃-Lösung gewaschen und getrocknet; die Ausbeute betrug 80 %.

Die Spaltung des DDP-OOH erfolgte derart, dass das DDP-OOH in eine gesättigte Lösung von Cu(OAC)₂ in Methanol (hergestellt aus etwa 94 mol% Cu(OAC)₂ und 12,3 Gewichtsteilen Methanol bezogen auf das DDP-OOH; die Konzentration an DDP-OOH in dieser Methanol-Menge lag bei etwa 0,25 mol/l) portionsweise eingebracht wurde. Es folgte die Zugabe von 2 Portionen FeSO₄ (je knapp 20 mol% bezogen auf DDP-OOH) und Rühren bei Umgebungstemperatur über Nacht. Zur Aufarbeitung wurde auf gesättigte wässrige NaCl-Lösung gegeben, mit Diisopropylether extrahiert und dieser Extrakt mit gesättigter wässriger NaHCO₃-Lösung und gesättigter wässriger NaCl-Lösung gewaschen. Nach Trocknung und fraktionierter Destillation wurden 73 % der Theorie an 11(12)-Pentadecen-15-oliden erhalten, die noch 8 % 15-Pentadecanolid enthielten.

In Russ. Chem. Bull. 1998, 47, 1166-1169 wurde DDP in 5,2 Gewichtequivalenten Eisessig bei 0°C vorgelegt und eine Mischung bestehend aus einer 50 %igen wässrigen Lösung von Schwefelsäure (etwa 26 mol% (=11 Gew.%) bezogen auf DDP) und 30 %igem Wasserstoffperoxid (etwa 1,89 molare Equivalente) zugegeben. Nach einer kurzen Nachreaktionszeit wurde der gebildete Feststoff (DDP-OOH) abfiltriert, dieser mit einer 50 %igen Essigsäurelösung (80 Gew.% bezogen auf DDP) und anschließend mehrfach mit Wasser (4 Waschvorgänge mit je 2 Gewichtsteilen Wasser bezogen auf DDP) bis zur Neutralität des Waschwassers gewaschen. Nach Trocknung des Feststoffes wurden 85 % der Theorie an DDP-OOH erhalten, das eine 96 %ige Reinheit aufwies.

Die Spaltung des DDP-OOH erfolgte derart, dass eine Suspension aus 1 Anteil DDP-OOH und etwa 3,8 Gewichtsanteilen 4-Methylpentan-2-on (MIBK) über einen längeren Zeitraum in eine siedende Lösung von Cu(OAC)₂ in etwa 3,8 Gewichtsanteilen MIBK (bezogen auf DDP-OOH) eindosiert wurde. Die Menge an Cu(OAC)₂ wurde im Bereich von 0,15 bis 7,0 mol%, bezogen auf das DDP-OOH, variiert, wobei bei 5 mol% Cu(OAC)₂ nach Angabe der Autoren das Optimum lag. Nach 3 Stunden der Nachreaktionszeit bei Siedehitze wurde das Reaktionsgemisch abgekühlt und von den ausgefallenen Kupfersalzen befreit. Das Filtrat wurde mit heißem Wasser (2 Waschvorgänge mit je 7,7 Gewichtsequivalenten Wasser bezogen auf DDP-OOH) gewaschen und eingeengt. Es wurde unter Verwendung von 5 mol% Cu(OAC)₂ eine Rohausbeute an 11 1(12)-Pentadecen-15-oliden von 96,5 % der Theorie erhalten.

Nachteile dieser Verfahren sind unter den Reaktionsbedingungen der Spaltung von DDP-OOH insbesondere das Ausfallen elementaren Kupfers und/oder unlöslicher Kupferverbindungen sowie die großen Mengen der in den Reaktionen verwendeten Reagenzien und Hilfsstoffe. Als weitere Nachteile sind beispielsweise die vielen, teils aufwendigen, Prozessschritte und Waschvorgänge zu nennen und die damit zusammenhängenden Umwelt-und Sicherheitsaspekte sowie schlechte Raum-Zeit-Ausbeuten. Bei einer Maßstabsvergrößerung des letztgenannten Verfahrens ist zudem nachteilig, dass erhebliche Mengen ringverengter Lactone mit einer ω-Hydroxyalkyl-Seitenkette gebildet werden.

Die bekannten Syntheseverfahren sind daher für eine industrielle Umsetzung ungeeignet. Ein technisches Verfahren, das auf einfache und kostengünstige Weise 11(12)-Pentadecen-15-olide liefert, ist deshalb von großem wirtschaftlichem Interesse.

Beschrieben wird nachfolgend auch ein Verfahren zur Herstellung von 11 (12)-Pentadecen-15-oliden ausgehend von DDP-OOH in Gegenwart einer Cu-(II)-verbindung und eines Verdünnungsmittels, dadurch gekennzeichnet, dass während der Reaktion ein Azeotrop enthaltend Wasser und Verdünnungsmittel abdestilliert wird.

Vorteilhafte Cu-(II)-verbindungen sind solche, die unter den Reaktionsbedingungen der DDP-OOH-Umsetzung in dem verwendeten Verdünnungsmittel löslich sind. Solche Cu-(II)-verbindungen weisen bei 20°C in dem Verdünnungsmittel eine Löslichkeit von mindestens 0,5 g/kg Verdünnungsmittel auf, bevorzugt von mindestens 1 g/kg. Die Cu-(II)-Verbindungen können wasserfrei oder als Hydrate (Kristallwasser) eingesetzt werden. Die Wassermenge des Kristallwassers ist unkritisch.

Bevorzugte Cu-(II)-verbindungen sind solche mit organischen Resten. Neben Cu-(II)-2,4-pentandionat-Derivaten sind dabei Cu-(II)-carboxylate besonders geeignet. Bevorzugte Cu-(II)-2,4-pentandionate sind Cu-(II)-acetylacetonat, Cu-(II)-1,1,1-trifluoracetylacetonat und [Bis(2,2,6,6-tetramethyl-3,5-heptandionato)]-Cu-(II). Besonders bevorzugt sind Cu-(II)-carboxylate von Alkylcarbonsäuren mit 2 bis 5 Kohlenstoffatomen, insbesondere Cu-(II)-acetat und Cu-(II)-propionat.

Es können ein oder mehrere Cu-(II)-verbindungen verwendet werden. Die vorteilhafte Menge der Cu-(II)-verbindungen bei der Umsetzung von DDP-OOH zu den 11(12)-Pentadecen-15-oliden beträgt 0,02 bis 1,5 mol% bezogen auf DDP-OOH, bevorzugt 0,05 bis 1 mol% und besonders bevorzugt 0,1 bis 0,6 mol%.

Als Verdünnungsmittel können alle flüssigen und mit Wasser Azeotrope bildenden organischen Verbindungen eingesetzt werden. Es fungieren diese Verdünnungsmittel bei der destillativen Entfernung des Verdünnungsmittels als Wasserschlepper. Es können ein oder mehrere Verdünnungsmittel verwendet werden, bevorzugt ist die Verwendung nur eines Verdünnungsmittels. Vorteilhafte Verdünnungsmittel haben bei Normaldruck einen Siedepunkt im Bereich 70 bis 230°C, besonders vorteilhaft im Bereich von 90 bis 150°C. Bevorzugte Verdünnungsmittel sind aliphatische Ester, Ether oder Ketone, insbesondere Ketone. Bevorzugter Ester ist Butylacetat, bevorzugter Ether ist Di-n-butylether Bevorzugte Ketone sind 2-Pentanon, 3-Pentanon, 4-Methylpentan-2-on (MIBK), Ethylisopropylketon und Diisopropylketon, besonders bevorzugt ist MIBK.

Es ist bevorzugt das bei der Spaltung abdestillierte Azeotrop vom Wasser zu befreien und das Verdünnungsmittel wieder für die Spaltung zu verwenden. Es ist dabei vorteilhaft, wenn dieses wiedergewonnene Verdünnungsmittel eine Säurezahl von kleiner oder gleich 3 mg KOH / g aufweist. Die Säurezahl entspricht der Anzahl der Milligramm an Kaliumhydroxid, die zur Neutralisation eines Gramms des wiedergewonnenen Verdünnungsmittels benötigt werden.

Das bevorzugte Gewichtsverhältnis von DDP-OOH zu der Gesamtmenge des in der Spaltung verwendeten Verdünnungsmittels liegt im Bereich 1 : 2 bis 1 : 7, bevorzugt bei 1 : 3 bis 1 : 5.

Der Temperaturbereich, in dem die Spaltung durchgeführt wird, liegt bei 70 bis 120°C. Bevorzugt wird die Spaltung bei Temperaturen im Bereich von 80 bis 100°C, besonders bevorzugt bei 85 bis 95°C durchgeführt.

Der vorteilhafte Druckbereich, in dem die Spaltung durchgeführt wird, liegt bei 0,01 mbar bis 2 bar. Bevorzugt wird das Verfahren bei Drücken unterhalb von 1013 mbar ausgeführt, insbesondere im Bereich von 50 bis 800 mbar. Wenn die Umsetzung diskontinuierlich erfolgt, so wird der Druck bevorzugt mit fortschreitender Spaltung immer weiter abgesenkt und die Temperatur der Spaltung weitgehend konstant gehalten, bevorzugt beginnend bei 450 bis 750 mbar und endend bei 100 bis 400 mbar.

Das in die Spaltung eingesetzte DDP-OOH kann wasserfrei oder wasserhaltig sein, bevorzugtes DDP-OOH ist wasserhaltig. Der Wassergehalt des DDP-OOH liegt vorteilhafterweise im Bereich von 5 bis 40 Gew.%, bevorzugt bei 10 bis 30 Gew.% und besonders bevorzugt bei 15 bis 25 Gew.%.

Überraschenderweise wurde zudem gefunden, dass bestimmte Additive ein Ausfallen von Kupfer und/oder unlöslichen Kupferverbindungen während der Spaltung unterdrücken. Bevorzugt wird die Umsetzung von DDP-OOH zu den 11(12)-Pentadecen-15-oliden in Gegenwart von Additiven durchgeführt, die durch folgende Formel wiedergegeben werden können:
- X und Y: unabhängig voneinander O oder N-R bedeuten, wobei R = H oder ein organischer Rest mit 1 bis 10 Kohlenstoffatomen ist, und
- A: ein organischer Rest umfassend 2 bis 100 Kohlenstoffatome ist.

Vorteilhafterweise liegt der Siedepunkt des Additivs oberhalb des Siedepunktes des verwendeten Verdünnungsmittels und oberhalb des azeotropen Gemisches von Verdünnungsmittel und Wasser.

Bevorzugt umfasst A 6 bis 50 Kohlenstoffatome, besonders bevorzugt 10 bis 30 Kohlenstoffatome. Der Rest R umfasst bevorzugt 1 bis 4 Kohlenstoffatome, bevorzugt ist R = Methyl oder Ethyl.

Der organische Rest A enthält bevorzugt die Heteroatome O oder N, bevorzugt in Form von Hydroxygruppen, Ethergruppen oder Aminogruppen, bevorzugt sind Ethergruppen und sekundäre Aminogruppen. An dem Kohlenstoffgerüst des Restes A können ein oder mehrere organische Gruppierungen angebracht sein, die unabhängig voneinander geradkettig, verzweigt, cyclisch, heterocyclisch, aromatisch oder heteroaromatisch sein können, wobei bei den heteroatomhaltigen Gruppierungen solche mit O oder N bevorzugt sind.

Vorteilhafte Additive sind α,ω-Diole und α,ω-Aminoalkohole.

In einer besonders vorteilhaften Ausfiihrungsform werden Additive eingesetzt, die als Heteroatome ausschließlich Sauerstoff enthalten. Diese α,ω-Diole enthalten im Kohlenstoffgerüst des organischen Restes A bevorzugt mindestens 2 Sauerstoffatome, bevorzugt in Form von Ethergruppen.

Besonders bevorzugte Additive sind Polyalkylenglykole, insbesondere Polyethylenglykole (PEG), Polypropylenglykole oder Polytetramethylenglykole (Polytetrahydrofurane), welche mindestens einen Polymerisationsgrad von 2 und vorzugsweise Besonders bevorzugte Additive sind Polyalkylenglykole, insbesondere Polyethylenglykole (PEG), Polypropylenglykole oder Polytetramethylenglykole (Polytetrahydrofurane), welche mindestens einen Polymerisationsgrad von 2 und vorzugsweise Molmassen im Bereich von 144 bis etwa 2200 aufweisen. Die Polyalkylenglykole sind bei höheren Molmassen polydispers und haben einen Molmassenbereich, z.B. weist PEG 1000 typischerweise einen Molmassenbereich von 950 bis 1050 auf. Ganz besonders bevorzugt sind Polyethylenglykole (PEG) mit Polymerisationsgraden von 4 bis 50. In besonderem Maße bevorzugt sind PEG 200 bis PEG 1000, hierbei wiederum PEG 400, PEG 600 und PEG 800. Diese Produkte sind handelsüblich verfügbar.

Es können ein oder mehrere Additive verwendet werden. Die vorteilhafte Menge der Additive bei der Umsetzung von DDP-OOH zu den 11(12)-Pentadecen-15-oliden beträgt 0,5 bis 15 Gew.% bezogen auf DDP-OOH, bevorzugt 1 bis 10 Gew.%, besonders bevorzugt 2 bis 8 Gew.% und ganz besonders bevorzugt 3 bis 6 Gew.%.

Die Spaltung wird bevorzugt dergestalt durchgeführt, dass eine Mischung enthaltend Verdünnungsmittel und Cu-(II)-verbindung sowie gegebenenfalls Additiv vorgelegt und aufgeheizt wird. Zu dieser geheizten Mischung wird dann eine Suspension oder Lösung enthaltend Verdünnungsmittel und DDP-OOH zugegeben. Es ist besonders vorteilhaft, die azeotrope destillative Entfernung des wasserhaltigen Verdünnungsmittels mit Dosierbeginn des DDP-OOH zu starten.

Nach beendeter Spaltung ist es vorteilhaft, die Kupferionen vor einer destillativen Isolierung der 11(12)-Pentadecen-15-olide weitestgehend zu deaktivieren. Hierzu ist es zweckdienlich, das Kupfer stark zu komplexieren und/oder als Kupferverbindung auszufällen. Hierzu sind insbesondere starke Komplexbildner des Kupfers geeignet, wie z.B. Kryptanden, EDTA oder Citronensäure.

Die Spaltung kann diskontinuierlich, semi-kontinuierlich oder kontinuierlich durchgeführt werden, bevorzugt ist die semi-kontinuierliche oder kontinuierliche Verfahrensführung.

Die Spaltung kann auch in einen kontinuierlich betriebenen Rührkesselreaktor ausgeführt werden. Es wird dann bevorzugt nicht nur eine Suspension aus DDP-OOH und Verdünnungsmittel dosiert, sondern parallel eine Suspension einer Cu-(II)-verbindung, Verdünnungsmittel sowie gegebenenfalls eines Additiv-Zusatzes. Man lässt die aus dem Rührkesselreaktor abgenommene rohe Suspension vorteilhafterweise noch durch einen zweiten kontinuierlich betriebenen Rührkesselreaktor, in dem weiteres wasserhaltiges Verdünnungsmittel abdestilliert wird, zur Nachreaktion laufen. Nach Bedarf kann in einem dritten kontinuierlichen betriebenen Rührkesselreaktor ein starker Cu-Komplexbildner eindosiert und die Destillation des wasserhaltigen Verdünnungsmittels fortgesetzt werden. Der Ablauf dieses Rührkesselreaktors kann auf eine kontinuierlich betriebene Destillationskolonne geleitet, bei der über Kopf Verdünnungsmittel und Reste an Wasser abgenommen werden und als Sumpfprodukt ein Verdünnungsmittel-freies Rohprodukt erhalten wird. Wurde ein starker Cu-Komplexbildner zugesetzt, so kann der gegebenenfalls ausgefallene Cu-Komplex abfiltriert werden und die rohen 11(12)-Pentadecen-15-olide weiter nach Bedarf gereinigt werden.

Typischerweise werden die 11(12)-Pentadecen-15-olide, sowohl bei kontinuierlich, semi-kontinuierlich oder diskontinuierlich durchgeführtem Verfahren, in einer isolierten Ausbeute von 97 % d.Th. bei der Spaltung und von 87 % d.Th. über alle Schritte ausgehend von DDP erhalten.

Gegenstand der vorliegenden Erfindung ist ein Verfahren zur Herstellung von DDP-OOH ausgehend von DDP und Wasserstoffperoxid in Gegenwart von Essigsäure und Schwefelsäure, dadurch gekennzeichnet, dass die Umsetzung in Gegenwart von 0,05 bis 5 mol% H₂SO₄ bezogen auf DDP durchgeführt wird.

Das erfindungsgemäß bevorzugte Gewichtsverhältnis von DDP zu Essigsäure liegt im Bereich 1 : 2 bis 1 : 8, bevorzugt bei 1 : 4 bis 1 : 6.

Für einen spontanen und gleichmäßigen Reaktionsverlauf ist es vorteilhaft, einen Teil des vorherigen Rohansatzes (nicht filtrierte rohe Reaktionsproduktmischung, gegebenenfalls noch DDP-OOH Kristalle enthaltend, deren Hauptbestandteil Essigsäure ist) vorzulegen.

Für die Addition von H₂O₂ an DDP zu DDP-OOH kann Wasserstoffperoxid unterschiedlichen Gehalts verwendet werden. Typischerweise wird 30 bis 70 %iges wässriges Wasserstoffperoxid eingesetzt, bevorzugt ist 50 oder 70%iges.

Die erfindungsgemäß bevorzugte Menge an H₂O₂ beträgt 0,8 bis 1,25 molare Equivalente bezogen auf DDP und besonders bevorzugt 0,95 bis 1,1 molare Equivalente.

Der Temperaturbereich, in dem die Addition von H₂O₂ durchgeführt wird, liegt bei 0 bis 10°C, bevorzugt bei 3 bis 8°C. Als im besonderen Maße geeignet erweist sich eine Reaktionstemperatur um 5°C.

Als Ausgangsmaterial für die Herstellung von DDP-OOH kann auch OCP verwendet werden. Hierbei ist es dann vorteilhaft mehr Essigsäure einzusetzen, typischerweise 10 bis 30 Gew.% mehr als bei der Verwendung von DDP als Edukt.

Nach beendeter Reaktion wird das Reaktionsgemisch filtriert und die DDP-OOH-Kristalle mit möglichst wenig Wasser gewaschen, um die Kristalle weitgehend von Essigsäure und Schwefelsäure zu befreien. Vorteilhaft ist eine zweifache Gegenstromwäsche mit Wasser, jeweils mit der doppelten Gewichtsmenge an Wasser bezogen auf DDP-OOH. Für diese Filtration eignen sich insbesondere Bandfilter und Stülpzentrifugen. Für die Waschvorgänge werden bevorzugt Wasser (z.B. demineralisert oder destilliert) und/oder Waschwässer vorheriger DDP-OOH-Kristallwäschen verwendet. Es ist besonders vorteilhaft, die nach Filtration und den Waschvorgängen vorliegenden DDP-OOH-Kristalle nicht weiter zu trocknen, sondern den erhaltenen Wassergehalt beizubehalten.

Das nach der Filtration der DDP-OOH-Kristalle und den sich anschließenden Waschvorgängen erhaltene Filtrat (kristallfreie Reaktionslösung) enthält hauptsächlich Essigsäure. Der Wassergehalt in diesem Filtrat beträgt meist 10 bis 25 Gew.%. Dieses Filtrat wird bevorzugt erneut für dieselbe Reaktion, zumeist nach Zugabe eines Anteils frischen Eisessigs, eingesetzt.

Die Waschvorgänge und die Filtration und die gegebenenfalls erfolgende Trocknung der gewaschenen DDP-OOH Kristalle sollten möglichst schonend und bei Temperaturen im Bereich von -10 bis +10°C erfolgen, bevorzugt ist eine Temperatur um 0°C.

Die nach Filtration, Waschvorgängen und gegebenenfalls Trocknung vorliegenden DDP-OOH-Kristalle weisen bevorzugt einen Wassergehalt von 5 bis 40 Gew.%, besonders bevorzugt von 10 bis 30 Gew.% und ganz besonders bevorzugt von 15 bis 25 Gew.% auf.

Die Herstellung von DDP-OOH kann diskontinuierlich, semi-kontinuierlich oder kontinuierlich durchgeführt werden.

Die Reaktion kann beispielsweise in einen kontinuierlich betriebenen Rührkesselreaktor ausgeführt werden. Es wird dann Wasserstoffperoxid einerseits und parallel eine Mischung enthaltend DDP, Essigsäure, Schwefelsäure und Wasser andererseits in diesen Rührkesselreaktor dosiert. Diese Mischung kann auch durch DDP, kristallfreie Reaktionslösung (z.B. aus dem Voransatz), etwas frische Schwefelsäure und frische Essigsäure und gegebenenfalls Wasser hergestellt werden. Lässt man die aus dem Rührkesselreaktor abgenommene Suspension noch durch einen zweiten kontinuierlich betriebenen Rührkesselreaktor zur Nachreaktion laufen, so unterscheidet sich die kontinuierliche Fahrweise nicht von der diskontinuierlichen (Batch) Variante in Bezug auf Ausbeute und Qualität des Produktes.

Typischerweise wird DDP-OOH, sowohl bei kontinuierlich, semi-kontinuierlich oder diskontinuierlich durchgeführtem Verfahren, in einer isolierten Ausbeute von 90 % d.Th. ausgehend von DDP erhalten.

Es ist empfehlenswert alle Schritte bei der Herstellung der 11(12)-Pentadecen-15-olide, insbesondere die Herstellung, Filtration und Handhabung der DDP-OOH-Kristalle, in einer Atmosphäre von Schutzgas oder Mischungen von Schutzgasen durchzufähren. Besonders geeignete Schutzgase sind beispielsweise Stickstoff, Argon, Helium oder Kohlendioxid.

Dass die Reaktion von DDP mit Wasserstoffperoxid zum DDP-OOH kontinuierlich, die Trennung von Reaktionslösung und DDP-OOH-Kristallen sowie deren Reinigung quasi kontinuierlich und die Umlagerung von DDP-OOH unter Kupfer-(II)-Katalyse zu den 11(12)-Pentadecen-15-oliden ebenfalls kontinuierlich ausgeführt werden kann, ist nicht nur unter ökonomischen Aspekten wichtig. Unter dem Aspekt von Sicherheit für Arbeit und Umwelt kann so die im Prozess befindliche Menge an Hydroperoxiden auf ein Miniumum verringert werden.

Mit dem vorliegenden Verfahren gelingt es, die Lösungsmittel- und Katalysator-Kreisläufe zu schließen bzw. die Abfälle auf ein Minimum zu reduzieren.

Die folgenden Beispiele erläutern die Erfindung.

### Beispiel 1

### Herstellung von DDP-OOH

In ein 6 L Doppelmantelgefäß mit Bodenablauf, versehen mit Blattrührer, 2 Dosierpumpen, Schlauchpumpe, Kryostat und Stickstoffanschluss wurden 448 g DDP, 2070 g Essigsäure, 330 g entmineralisiertes Wasser und 4 g Schwefelsäure auf 5°C thermostatisiert vorgelegt. Es wurde mit 400 UpM (Umdrehungen pro Minute) gerührt. Innerhalb von 30 Minuten wurden 140 g 50 %iges Wasserstoffperoxid dosiert. Die Reaktion war exotherm und spontan. Nach 15 Minuten Nachreaktionszeit wurde die Reaktionsmischung über eine Porzellan-Nutsche filtriert und zweimal mit je 1000 g Wasser gewaschen (die vereinigten Filtrate bilden die kristallfreie Reaktionslösung). Die Ausbeute an DDP-OOH (ohne Wassergehalt von etwa 17 Gew.%) lag bei 89 % der Theorie.

### Beispiel 2

### Herstellung von DDP-OOH

In ein 6 L Doppelmantelgefäß mit Bodenablauf, versehen mit Blattrührer, Dosierpumpe, Kryostat und Stickstoffanschluss wurden 2600 g kristallfreie Reaktionslösung aus Voransatz (aus Beispiel 1), 448 g DDP, 180 g Essigsäure und 0,4 g Schwefelsäure auf 5°C thermostatisiert vorgelegt. Es wurde mit 400 UpM gerührt. Innerhalb von 30 Minuten wurden 100 g 70 %iges Wasserstoffperoxid dosiert. Die Reaktion war exotherm und spontan. Die Nachreaktionszeit betrug 15 Minuten. 300 g der rohen Reaktionsproduktmischung verblieben für den Folgeansatz im Reaktor. Die übrigen 3000 g wurden der weiteren Aufreinigung zugefuhrt.

### Beispiel 3

### Kontinuierliche Herstellung von DDP-OOH

In ein 6 L Doppelmantelgefäß mit Bodenablauf ausgestattet mit Blattrührer, 2 Dosierpumpen, Schlauchpumpe, Kryostat und Stickstoffanschluss wurden 300 g des vorherigen Rohansatzes (nicht filtrierte rohe Reaktionsproduktmischung, noch DDP-OOH Kristalle enthaltend), 448 g DDP, 2070 g Essigsäure, 330 g entmineralisiertes Wasser und 4 g Schwefelsäure auf 5°C thermostatisiert vorgelegt. Es wurde mit 400 UpM gerührt. Innerhalb von 30 Minuten wurden 140 g 50 %iges Wasserstoffperoxid eindosiert. Die Reaktion war exotherm und spontan. Nach 15 Minuten wurde die Reaktion kontinuierlich fortgesetzt, indem mit zwei Dosierpumpen 70 %iges Wasserstoffperoxid in der einen Pumpe und ein Dosiergemisch aus kristallfreier Reaktionslösung, DDP, Essigsäure und Schwefelsäure in der anderen Pumpe parallel in das Reaktionsgefäß eingebracht wurde. Gleichzeitig wurde mittels einer Schlauchpumpe über den Bodenablass Reaktionslösung abgenommen. Es wurde Wasserstoffperoxid 70 %ig mit einer Geschwindigkeit von 200 g/h dosiert. Das Dosiergemisch wurde in einem Verhältnis von 2300 g kristallfreier Reaktionslösung, 448 g DDP, 180 g Essigsäure und 0,4 g Schwefelsäure angesetzt und mit einer Geschwindigkeit von 5857 g/h eindosiert. Die Reaktionslösung wurde mit einer Geschwindigkeit von etwa 6057 g/h derart entnommen, dass das Flüssigkeitsniveau im Reaktor weitgehend konstant blieb. Der Rohansatz wurde der Filtration und der weiteren Aufreinigung zugeführt.

### Beispiel 4

### Filtration von DDP-OOH

Zur Trennung der DDP-OOH Kristalle von ihrer Mutterlauge wurde eine Stülpzentrifuge genutzt. Diese wurde vor Beginn des Schleuderprozesses auf etwa +5°C gekühlt und mit Stickstoff inertisiert. Die Zentrifuge wurde auf eine Schleuderdrehzahl von 2300 UpM eingestellt. 15 kg der Reaktionslösung wurden mit einer Schlauchpumpe innerhalb von 1 bis 1,5 Minuten eingepumpt. Die Dosierleitung wurde mit 2 kg kristallfreier Reaktionslösung nachgespült. Für 5 Minuten wurde mit 2300 UpM geschleudert. Es fielen 13,9 kg kristallfreier Reaktionslösung an, die bis zum Wiedereinsatz auf 1 °C thermostatisiert wurden. Nach Reduzierung der Schleuderdrehzahl auf 600 UpM wurde die Zentrifuge durch Stülpen ausgeräumt. Man erhielt 3,1 kg feuchte Kristalle an DDP-OOH.

Die Kristalle wurden bei etwa 1°C mit 6,9 kg Waschwasser angemaischt und 15 Minuten kräftig gerührt. Als Waschwasser kann entmineralisiertes Wasser oder das Filtrat vorheriger Filtrationsansätze eingesetzt werden (Essigsäure-Gehalt etwa 3 Gew.%).

Die Stülpzentrifuge wurde auf eine Schleuderdrehzahl von 2600 UpM eingestellt. Mit einer Schlauchpumpe wurden 10 kg DDP-OOH-Suspension wurden innerhalb von 1 Minute eindosiert. Es wurde eine weitere 1 Minute mit unveränderter Drehzahl zwischengeschleudert. Es fielen 6,1 kg Abwasser an, das verworfen wurde. Bei gleichbleibender Drehzahl wurde mit 5,9 kg auf 1°C vorgekühltem entmineralisierten Wasser, das innerhalb von 1 Minute mit einer Dosierpumpe eingepumpt wurde, gewaschen. Es wurde danach 10 Minuten lang bei 2600 UpM geschleudert. Es fielen 6,9 kg Waschwasser an (die im nachfolgenden Ansatz wieder eingesetzt werden). Nach beenden des Schleuderns wurde die Schleuderdrehzahl auf 600 UpM reduziert. Die Zentrifuge wurde durch Stülpen ausgeräumt. Es wurden 2,9 kg DDP-OHH-Kristalle mit einem Feuchtegehalt von ca. 20 % erhalten, was einer Menge von 2,30 kg DDP-OOH entspricht. Die Ausbeute lag somit bei 90 % d.Th.. bezogen auf DDP umfassend die Stufen DDP-OOH-Bildung und dessen Isolierung. Das Produkt war praktisch frei von Essigsäure und Schwefelsäure.

### Beispiel 5

### Herstellung von 11 (12)-Pentadecen-15-oliden (nicht erfindungsgemäß)

In ein mit Stickstoff inertisiertes 6 L Doppelmantelgefäß ausgestattet mit Rührer, Thermostat, Destillationskolonne, Kolonnenkopf, Vakuumpumpe und Vakuumregelung wurde eine Mischung aus 800 g MIBK 100 g PEG 400 und 3,6 g Kupfer-IIacetat-Monohydrat vorgelegt. Es wurde aufgeheizt und 30 Minuten lang unter Rückfluss gekocht (Kopftemperatur etwa 112°C, Sumpftemperatur etwa 118°C). Es wurde vorsichtig ein Vakuum von 550 mbar eingeregelt, so dass sich eine Sumpf-temperatur von 90°C und eine Kopftemperatur von 85°C einstellte.

2,9 kg des DDP-OOH (feucht, ca. 20 Gew.% Wasser, aus Beispiel 4) und 9 kg MIBK (gegebenenfalls aus der DDP-OOH-Spaltung wiedergewonnenes MIBK) wurden in einer auf 0°C thermostatisierten Dosiervorlage mit Rührer angemaischt. Aus der Dosiervorlage wurden von dieser Suspension etwa 2 L/h mittels einer Schlauchpumpe in das 6 L Doppelmantelgefäß eingebracht. Mit Beginn der Dosierung wurde am Kolonnenkopf etwas weniger an MIBK abgenommen als mit der Suspension in das Doppelmantelgefäß eindosiert wurde. Es wurde solange azeotrop abdestilliert, bis sich kein Wasser mehr abscheidet. So waren am Ende der Dosierung 8 kg MIBK (Säurezahl 2,0 mg KOH/g) und etwa 600 g Wasser abdestilliert. Das MIBK kann vorteilhafterweise wiederverwendet werden. Im Laufe der Dosierung wurde, um die Sumpftemperatur bei etwa 90°C zu halten, das Vakuum auf etwa 350 mbar abgesenkt. Dabei fiel die Kopftemperatur bis auf etwa 70°C.

Nach Abbruch der Destillation wurde die Heizung abgestellt und mit Stickstoff begast. Nach Zugabe von 5 g Citronensäure wurde unter Rühren bei Normaldruck aufgeheizt und solange destilliert, bis sich kein Wasser mehr abscheidet. Die Sumpftemperatur lag dann bei ca. 120°C und die Kopftemperatur bei etwa 115°C. Unter vorsichtigem Anlegen von Vakuum wurde die Destillation fortgesetzt. Bei einer Sumpftemperatur von 120°C und einem Vakuum von etwa 60 mbar wurde die Destillation abgebrochen und mit Stickstoff begast. Das erhaltene Destillat bestand aus etwa 30 g Wasser und 1,6 kg MIBK. Bei 50°C bis 60°C wurde das Rohprodukt über eine Porzellan-Nutsche filtriert und zweimal mit 100 g MIBK gewaschen. Das Filtrat und die beiden Waschfiltrate wurden getrennt aufgefangen. Es wurden 2250 g Filtrat und 6,2 g trockenes Kupfercitrat sowie ca. 250 g Waschfiltrate erhalten. Durch einengen können aus den Waschfiltraten weitere 50 g Filtrat gewonnen werden.

Nach erneuter Destillation wurden 40 g Vorläufe, 2080 g 11 (12) -Pentadecen- 15 -olide und 150 g Destillationsrückstände, die unter anderem das PEG 400 enthalten, erhalten. Das entspricht einer Ausbeute an 11(12)-Pentadecen-15-oliden von 87 % erhalten. Das entspricht einer Ausbeute an 11(12)-Pentadecen-15-oliden von 87 % d.Th. über alle Schritte ausgehend von DDP und einer Ausbeute von 97 % d.Th. für den Schritt vom DDP-OOH zu den 11(12)-Pentadecen-15-oliden.

### Beispiel 6

### Kontinuierliche Herstellung von 11(12)-Pentadecen-15-oliden (nicht erfindungsgemäß)

Es wird wie in Beispiel 5 verfahren, bis zum Ende der Dosierung und dem Punkt der azeotropen Destillation, an dem 8 kg MIBK und etwa 600 g Wasser abdestilliert worden waren.

An diesem Punkt wurde die Reaktion kontinuierlich fortgesetzt, indem weiterhin über die Schlauchpumpe eine DDP-OOH-Suspension, über eine zweite Dosierpumpe ein zweites Dosiergemisch eindosiert und über eine dritte Schlauchpumpe über den Bodenablass fortlaufend Reaktionslösung entnommen wurde. Die DDP-OOH-Suspension wurde in einem Verhältnis von 2,9 kg DDP-OOH (feucht, ca. 20 Gew.% Wasser, aus Beispiel 4) zu 9 kg MIBK angesetzt und mit einer Dosiergeschwindigkeit von 1,7 kg/h dosiert. Das zweite Dosiergemisch wurde aus 800 g MIBK, 100 g PEG 400 und 3,6 g Cu(OAc)₂-Monohydrat angesetzt und als sehr gut gerührte, auf 110°C vorgeheizte Suspension verwendet. Dieses zweite Dosiergemisch wurde mit einer Dosiergeschwindigkeit von 130 g/h dosiert. Über den Kolonnenkopf wurden 1230 g wasserhaltiges MIBK pro Stunde abdestilliert. Über den Bodenablass wurden 600 g/h Reaktionslösung abgeführt.

Die weitere Aufarbeitung der Reaktionslösung erfolgte entsprechend Beispiel 5 mit den gleichen Ergebnissen hinsichtlich der Ausbeuten.

## Patentansprüche

1. Verfahren zur Herstellung von 1-Hydroperoxy-16-oxabicyclo[10.4.0]hexa-decan (DDP-OOH) ausgehend von 13-Oxabicyclo[10.4.0]hexadec-1(12)-en (DDP) und Wasserstoffperoxid in Gegenwart von Essigsäure und Schwefelsäure, **dadurch gekennzeichnet, dass** die Umsetzung in Gegenwart von 0,05 bis 5 mol% H₂SO₄ bezogen auf DDP durchgeführt wird.

2. Verfahren nach Anspruch 1, **dadurch gekennzeichnet, dass** die Menge an H₂SO₄ 0,1 bis 3 mol% bezogen auf DDP beträgt.

3. Verfahren nach Anspruch 1 oder 2, **dadurch gekennzeichnet, dass** 0,8 bis 1,25 molare Equivalente, bezogen auf DDP, an Wasserstoffperoxid verwendet werden.

4. Verfahren nach mindestens einem der Ansprüche 1 bis 3, **dadurch gekennzeichnet, dass** nach beendeter Reaktion eine Filtration und Waschvorgänge angeschlossen werden.

5. Verfahren nach mindestens einem der Ansprüche 1 bis 4, **dadurch gekennzeichnet, dass** zumindest zum Teil Waschwässer vorheriger Ansätze verwendet werden.

6. Verfahren nach mindestens einem der Ansprüche 1 bis 5, **dadurch gekennzeichnet, dass** der Wassergehalt des DDP-OOH im Bereich von 5 bis 40 Gew.% liegt.

7. Verfahren nach Anspruch 1 oder 2, **dadurch gekennzeichnet, dass** das Gewichtsverhältnis von DDP zu Essigsäure im Bereich von 1:2 bis 1:8 liegt.

## Claims

1. Method for producing 1-hydroperoxy-16-oxabicyclo[10.4.0]hexadecane (DDP-OOH) starting from 13-oxabicyclo[10.4.0]hexadec-1(12)-ene (DDP) and hydrogen peroxide in the presence of acetic acid and sulfuric acid, **characterized in that** the reaction in carried out in the presence of 0.05 to 5 mol% H₂SO₄ with respect to DDP.

2. Method according to claim 1, **characterized in that** the amount of H₂SO₄ is 0.1 to 3 mol% with respect to DDP.

3. Method according to claim 1 or 2, **characterized in that** 0.8 to 1.25 molar equivalents with respect to DDP of hydrogen peroxide are used.

4. Method according to at least one of claims 1 to 3, **characterized in that** after the reaction is finished, a filtration and washing procedures follow.

5. Method according to at least one of claims 1 to 4, **characterized in that** at least partially washing waters of previous processes are used.

6. Method according to at least one of claims 1 to 5, **characterized in that** the water content of DDP-OOH is in the range of 5 to 40 wt.-%.

7. Method according to claim 1 or 2, **characterized in that** the weight ratio of DDP to acetic acid is in the range from 1:2 to 1:8.

## Revendications

1. Procédé pour la préparation de 1-hydroperoxy-16-oxabicyclo-[10.4.0]hexadécane (DDP-OOH) à partir de 13-oxabicyclo[10.4.0]hexadec-1(12)-ène (DDP) et de peroxyde d'hydrogène en présence d'acide acétique et d'acide sulfurique, **caractérisé en ce que** la réalisation est effectuée en présence de 0,05 à 5 mol% de H₂SO₄ par rapport au DDP.

2. Procédé selon la revendication 1, **caractérisé en ce que** la quantité de H₂SO₄ s'élève à 0,1 à 3 mol% par rapport au DDP.

3. Procédé selon la revendication 1 ou 2, **caractérisé en ce que** 0,8 à 1,25 équivalent molaire de peroxyde d'hydrogène par rapport au DDP est utilisé.

4. Procédé selon au moins l'une des revendications 1 à 3, **caractérisé en ce que**, une fois la réaction terminée, une filtration et des opérations de lavage sont effectuées.

5. Procédé selon au moins l'une des revendications 1 à 4, **caractérisé en ce que** des eaux de lavage des mélanges précédents sont utilisées au moins partiellement.

6. Procédé selon au moins l'une des revendications 1 à 5, **caractérisé en ce que** la teneur en eau du DDP-OOH se situe dans la plage de 5 à 40 % en poids.

7. Procédé selon la revendication 1 ou 2, **caractérisé en ce que** le rapport des poids du DDP et de l'acide acétique se situe dans la plage de 1:2 à 1:8.
